# EUROPÄISCHE PATENTSCHRIFT

(11) **EP 2 228 013 B1**
(45) Veröffentlichungstag und Bekanntmachung des Hinweises auf die Patenterteilung: **13.04.2016**
(21) Anmeldenummer: 10153336.2
(22) Anmeldetag: 11.02.2010
(51) Int. Cl.: A61B 8/12, A61B 8/06, A61B 8/08, G01S 15/58, G01S 13/58, G01S 15/89

(54) **Verfahren zur simultanen Bestimmung von Position und Geschwindigkeit von Objekten**
Method for simultaneous determination of the position and speed of objects
Procédé de détermination simultanée de la position et de la vitesse d'objets

(30) Priorität: 13.03.2009 DE 102009012821
(43) Veröffentlichungstag der Anmeldung: 15.09.2010
(73) Patentinhaber: Wrobel, Miroslaw, 97753 Karlstadt (DE)
(72) Erfinder: Wrobel, Miroslaw, 97753 Karlstadt (DE)
(74) Vertreter: von den Steinen, Axel

(56) Entgegenhaltungen:
- WO-A1-02/086537
- WO-A2-2005/098471
- US-A1- 2005 101 868
- US-A1- 2009 036 780

## Beschreibung

Die Erfindung betrifft ein Verfahren nach Anspruch 1.

Verfahren zur Bestimmung der Geschwindigkeit und der Position von sich bewegenden Medien bzw. Objekten sind im Stand der Technik bekannt. So werden im Bereich der elektromagnetischen Wellen mit Radarsystemen beispielsweise im Luftfahrtbereich Position und Geschwindigkeit von Flugzeugen bestimmt und weiterhin im Medizinbereich mittels Ultraschallwellen die Tiefenlage von Objekten und Blutgefäßen sowie Strömungsgeschwindigkeit von fließendem Blut bestimmt.

Die WO 02/086537 A1 und die WO 2005/098471 A2 beschreiben Verfahren zur simultanen Bestimmung von Position und Geschwindigkeit von Objekten durch Erzeugung eines Signals und Auswertung des am Objekt reflektierten Echosignals.

Auch sind zwar Ansätze zur simultanen Geschwindigkeitsbestimmung und Positionsbestimmung bekannt, diese unterliegen aber erheblichen Beschränkungen bezüglich Auflösung und Messbarkeit der Geschwindigkeit. Für eine bessere Auflösung ist es nämlich beispielsweise beim medizinischen Ultraschall-PW-Verfahren erforderlich, die ausgesendeten Wellenpakete möglichst klein zu halten (die Auflösungsgrenze entspricht dem halben Wellenpaket). Dies führt jedoch wiederum zu einer schlechteren Detektierbarkeit der Wellenverschiebung, verursacht durch den Dopplereffekt und somit zu einer Verschlechterung der Bestimmung der Geschwindigkeit.

Weiterhin ist beispielsweise das Ultraschall-CW-Verfahren für eine relativ genaue Bestimmung der Flussgeschwindigkeit bekannt. Jedoch lässt es keinen Rückschluss auf die Tiefe des Flusses zu.

Ebenso nachteilig ist es, dass beim Aussenden von Pulsen, beispielsweise beim Ultraschall-PW-Verfahren oder beispielsweise bei Kombination verschiedener Verfahren, jeweils abgewartet werden muss, bis alle auf den gesendeten Puls folgenden Echos abgeklungen sind, um eine eindeutige Bestimmung von Tiefenlage bzw. Position des Echoursprungs zu gewährleisten.

Folglich ist es bisher erforderlich, eine Wartezeit zwischen dem Absetzen verschiedener Messpulse abzuwarten, was die Gesamtzeit der Messung verlängert, oder auf Genauigkeit bei der Bestimmung der Tiefe bzw. Position und bei der Bestimmung der Geschwindigkeit des fließenden Mediums zu verzichten.

Darüber hinaus kann derzeit bei gleichzeitiger Bestimmung von Lage und Geschwindigkeit mittels beispielsweise des gepulsten Ultraschall-PW-Verfahrens die Geschwindigkeit des fließenden Mediums nicht kontinuierlich, sondern nur zeitlich beabstandet in Intervallen bestimmt werden, wodurch beispielsweise in den Pausenzeiten keine Verfolgung des Geschwindigkeitsverlaufs möglich ist.

Diese Probleme und Nachteile treten sowohl im Ultraschallbereich als auch im Bereich von elektromagnetischen Wellen auf.

Diese Einschränkungen sind vor allem im Bereich der Zugangslegung in Blutgefäßen oder bei der Perikardpunktion nachteilig. Der Zugang dient in erster Linie zur intravasalen, kontinuierlichen Blutdruckmessung, zur Entnahme von Blut für Labor- und Blutgasanalysen und zum Einführen von Instrumenten (beispielsweise bei Herzkatheter-Untersuchungen).

Das Vorschieben eines Führungsdrahtes eines Katheters erfolgt häufig unter Durchleuchtung, um die Position des Drahtes verfolgen zu können. Dabei unterliegt der Patient aber einer Strahlenbelastung. Anschließend wird unter Fixierung des Führungsdrahtes die Punktionskanüle entfernt. Dabei ist das Blutgefäß an der Einstichstelle zu komprimieren und streng darauf zu achten, dass die Position des Drahtes unverändert bleibt. Je nach Kaliber des einzubringenden Katheters (bzw. Drains oder der Schleuse) muss zuvor mittels Dilatator der Stichkanal aufgedehnt werden, um die Einführung zu erleichtern. Danach wird die Schleuse oder der Katheter über den Draht an seine Zielposition vorgeschoben. Während dessen ist es von Vorteil, sowohl die Blutflussgeschwindigkeit als auch die Position des Drahtes gleichzeitig zu überwachen, um beispielsweise einen Kollaps des Blutgefäßes zu verhindern.

Im Stand der Technik ist kein Verfahren bekannt, das gleichzeitig die Überwachung der Blutflussgeschwindigkeit sowie der Position des Führungsdrahtes oder auch des Blutgefäßes per Ultraschall ermöglicht.

Es ist daher die Aufgabe der vorliegenden Erfindung, ein Verfahren zur simultanen Bestimmung von Position und Geschwindigkeit von Objekten vorzuschlagen, welches eine bessere Auflösung bietet, mit geringerem baulichem Aufwand auskommt und eine simultane Messung von sowohl Geschwindigkeit als auch Position ermöglicht.

Diese Aufgabe wird durch die vorliegende Erfindung nach der Lehre des Hauptanspruchs gelöst.

Vorteilhafte Ausführungsformen der Erfindung sind Gegenstand der Unteransprüche.

Erfindungsgemäß werden die Nachteile dadurch überwunden, dass das Verfahren zur simultanen Bestimmung von Position und Geschwindigkeit von Objekten folgende Schritte umfasst:
- simultane Erzeugung eines ersten Signals zur Geschwindigkeitsbestimmung und eines zweiten Signals zur Positionsbestimmung,
- Mischen des ersten Signals mit dem zweiten Signal zu einem Mischsignal und anschließendes Senden des Mischsignals mittels eines Senders in Richtung eines Objektes, sodass das Mischsignal zumindest teilweise an dem Objekt reflektiert und als Echosignal empfangen wird,
- Auswertung des Echosignals .

Die Erzeugung der Signale kann beispielsweise mittels Signalgeneratoren oder dergleichen erfolgen, wobei die Signale in bevorzugter Weise separat generiert werden. Dabei kann es sich beim ersten Signal (im Folgenden auch als Geschwindigkeitssignal bezeichnet) beispielsweise um ein CW-Signal handeln und beim zweiten Signal (im Folgenden auch als Positionssignal bezeichnet) beispielsweise um ein korrelationsgünstig moduliertes Signal handeln.

Dann werden das Geschwindigkeitssignal und das Positionssignal zunächst zu einem Mischsignal gemischt, und danach wird das Mischsignal über einen Sender in Richtung eines zu untersuchenden Objektes gesendet.

Dabei können beispielsweise die Signale nach ihrer Erzeugung und gegebenenfalls Aufbereitung an ein Mischglied geleitet werden, welches das Geschwindigkeitssignal und das Positionssignal zu einem Mischsignal verarbeitet, und danach wird dieses eine Mischsignal über einen Sender in das Übertragungsmedium eingekoppelt. Dabei findet die Mischung des Geschwindigkeitssignals und des Positionssignals bereits vor dem Senden statt.

Gegenüber einer ebenfalls möglichen Variante mit zwei verschiedenen Sendern zum getrennten Senden des Geschwindigkeitssignals bzw. des Positionssignals ohne vorherige Mischung erfordert das Verfahren weniger baulichen Aufwand, da in diesem Beispiel auf einen Sender verzichtet werden kann. Darüber hinaus kann die Genauigkeit damit erhöht werden, weil Gangunterschiede durch beabstandete Sender oder deren räumliche Inkohärenz vermieden werden können.

Durch die Sendung eines Mischsignals, welches die Information sowohl des ersten Signals als auch des zweiten Signals enthält, kann eine simultane Bestimmung von sowohl Geschwindigkeit als auch Position ermöglicht werden. Es gelangen nämlich beide Signale, das erste Signal und das zweite Signal, gleichzeitig - und nicht zeitlich getrennt - zum Objekt.

Durch das simultane vermischte Senden sowohl des Geschwindigkeitssignals als auch des Positionssignals als Mischsignal lassen sich auch zwei physikalische Größen, beispielsweise Geschwindigkeit und Position, zeitgleich kontinuierlich bestimmen. Damit können Totzeiten bei der Bestimmung von physikalischen Größen vermieden werden.

Dann wird das Mischsignal über ein Übertragungsmedium (beispielsweise Schallwellen leitende Materie, Luft oder im Fall der elektromagnetischen Wellen auch ein Vakuum) in Richtung des zu untersuchenden Objekts ausgestrahlt.

Erfindungsgemäß umfasst die Auswertung eine Durchführung einer Geschwindigkeitsauswertung des Echosignals unter Berücksichtigung des ersten Signals und des Dopplereffektes, wobei als ein Ergebnis dieser Geschwindigkeitsauswertung eine Geschwindigkeitsinformation des Objektes erhalten wird.

Dabei kann beispielsweise bei der Geschwindigkeitsauswertung das Echosignal unter Berücksichtigung des Geschwindigkeitssignals und des Dopplereffekts ausgewertet werden. Durch die Berücksichtung des Geschwindigkeitssignals bei der Auswertung des Echosignals und des Dopplereffekts kann der Informationsgehalt vom Geschwindigkeitssignal aus dem Echosignal herausgetrennt werden, dabei durch mathematische Operationen Einfluss auf die Genauigkeit und Auflösung genommen werden und als ein Ergebnis dieser Geschwindigkeitsauswertung eine Geschwindigkeitsinformation des Objekts erhalten werden.

Erfindungsgemäß umfasst die Auswertung außerdem eine Durchführung einer Positionsauswertung des Echosignals unter Berücksichtigung des zweiten Signals, wobei als ein Ergebnis dieser Positionsauswertung eine Positionsinformation des Objektes erhalten wird.

Bei der Durchführung der Positionsauswertung des Echosignals kann beispielsweise das Positionssignal berücksichtigt und damit der Informationsgehalt des Positionssignals aus dem Echosignal herausgetrennt werden, wobei dabei durch mathematische Operationen Einfluss auf die Genauigkeit und Auflösung genommen werden kann. Als Ergebnis dieser Positionsauswertung wird eine Positionsinformation des Objektes erhalten.

Das Echosignal wird dadurch Auswertungen unterzogen, um an die physikalischen Größen zu gelangen. Damit sind beide Informationen, die Geschwindigkeitsinformation und die Positionsinformation, simultan und kontinuierlich erhältlich.

In einer bevorzugten Ausführungsform umfasst das Verfahren weiterhin einen kontinuierlichen Empfang eines vom Objekt reflektierten Echosignals.

Der Einsatzbereich des Verfahrens ist bezüglich des Signalbereichs beliebig. In einer bevorzugten Ausführungsform können die Signale in Form von Ultraschallwellen gebildet werden, und in einer weiteren bevorzugten Ausführungsform können die Signale in Form von elektromagnetischen Wellen gebildet werden.

In einer weiteren bevorzugten Ausführungsform ist das erste Signal ein dopplergünstiges Signal, d.h. es ist gut geeignet, um die Frequenzverschiebung zu bestimmen.

In einer weiteren bevorzugten Ausführungsform ist das zweite Signal von einem im Sinne der Mustererkennung positionsgünstigen Signaltyp, d.h. es ist gut geeignet, bei Auswertungen Muster wieder zu erkennen.

Die Form der verwendeten Signale ist grundsätzlich beliebig. In einer weiteren bevorzugten Ausführungsform weist das zweite Signal eine konstante Frequenz auf und wird in Intervallen gesendet. Damit ist das Positionssignal einfach generierbar, und durch die Sendung in Intervallen wird dem Signal ein Muster aufgeprägt, durch das beispielsweise über Betrachtung von Echolaufzeiten ein Rückschluss über die Position des Objekts möglich ist.

Die Form bezüglich des Verlaufs des Positionssignals ist grundsätzlich beliebig. Es hat sich als vorteilhaft erwiesen, wenn das zweite Signal einer mathematisch korrelierbaren Funktion folgt, um die unten dargestellte verbesserte Auflösungsmöglichkeit zu unterstützen. Je besser die Korrelation ist, desto besser kann auch die Auflösung bei der Positionsbestimmung werden.

In einer weiteren vorteilhaften Ausführungsform weist das zweite Signal eine zeitlich modulierte Frequenz (FM) auf. Dadurch wird dem Positionssignal ebenfalls ein Muster aufgeprägt, durch welches ebenfalls ein Rückschluss auf die Echolaufzeiten und somit die Position des Objekts möglich wird.

In einer weiteren vorteilhaften Ausführungsform folgt das Mischsignal beispielsweise einer im mathematischen Sinne glatten Funktion. Dies bietet insbesondere im Ultraschallbereich Vorteile, weil dadurch Ansprechverzögerungen bei beispielsweise Piezo-Ultraschallerzeugern vermieden werden können.

Bei der Wahl des Geschwindigkeitssignals hat es sich als vorteilhaft erwiesen, das Signal kontinuierlich mit einer konstanten, nicht veränderten Frequenz auszusenden, um anhand dessen die Dopplerverschiebung gut messen zu können. Die Frequenz des Geschwindigkeitssignals sollte sich dabei nicht mit der bzw. den Frequenz(en) im Positionssignal überschneiden.

Bei einer weiteren bevorzugten Ausführungsform umfasst die Positionsauswertung eine Filterung des Echosignals bezüglich der Frequenz des zweiten Signals. Dadurch kann die darauf folgende Auswertung beispielsweise durch geringeres Rauschen verbessert werden.

Bei einer weiteren bevorzugten Ausführungsform umfasst die Positionsauswertung eine Analyse der Dopplerverschiebung und/oder der Echolaufzeit. Dabei wird das ggf. zuvor gefilterte Echosignal einer Analyse unterzogen. Diese Analyse kann unter anderem entweder die Betrachtung der Dopplerverschiebung oder der Echolaufzeit oder beides umfassen.

Bei einer weiteren vorteilhaften Ausführungsform umfasst die Positionsauswertung eine Korrelation des Echosignals mit dem zweiten Signal, und anschließend wird eine Analyse der Echolaufzeit durchgeführt.

Über die Güte der Korrelation kann entscheidender Einfluss auf die Auflösung bezüglich der Positionsbestimmung genommen werden und diese damit erheblich verbessert werden. Alternativ zur Korrelation kann die Faltung oder auch eine andere Methode zur Mustererkennung angewendet werden.

In einer bevorzugten Ausführungsform wird das beschriebene Verfahren zur Überwachung der Zugangslegung in Blutgefäßen und/oder zur Perikardpunktion verwendet.

In einer weiteren bevorzugten Ausführungsform wird das beschriebe Verfahren zur Bestimmung von Blutflussgeschwindigkeit und Position von Blutgefäßen verwendet.

Mehrere Ausführungsformen der Erfindung sind in den Zeichnungen dargestellt und werden nachfolgend beispielhaft erläutert.

Es zeigen:
- Fig. 1: ein Geschwindigkeitssignal mit konstanter Frequenz gemäß einer ersten Ausführungsform;
- Fig. 2: ein in Intervallen gesendetes Positionssignal mit konstanter Frequenz gemäß einer ersten Ausführungsform;
- Fig. 3: ein Mischsignal aus dem Geschwindigkeitssignal gemäß Fig. 1 und dem Positionssignal gemäß Fig. 2;
- Fig. 4: ein nach dem Absenden des Mischsignals gemäß Fig. 3 empfangenes Echosignal;
- Fig. 5: ein bezüglich der Frequenz des Geschwindigkeitssignals gemäß Fig. 1 gefiltertes Echosignal gemäß Fig. 4;
- Fig. 6: ein gemäß der Frequenz des Positionssignals gemäß Fig. 2 gefiltertes Echosignal gemäß Fig. 4;
- Fig. 7: ein mit dem Positionssignal gemäß Fig. 2 korreliertes Echosignal gemäß Fig. 4;
- Fig. 8: ein Geschwindigkeitssignal mit konstanter Frequenz gemäß einer zweiten Ausführungsform;
- Fig. 9: ein Positionssignal mit modulierter Frequenz gemäß einer zweiten Ausführungsform;
- Fig. 10: eine beispielhafte Modulationsfunktion der Frequenzmodulation des Positionssignals gemäß Fig. 9;
- Fig. 11: ein Mischsignal aus dem Geschwindigkeitssignal gemäß Fig. 8 und dem Positionssignal gemäß Fig. 9;
- Fig. 12: ein nach dem Senden des Mischsignals gemäß Fig. 11 erhaltenes Echosignal;
- Fig. 13: ein Echosignal gemäß Fig. 12 korreliert mit dem Positionssignal gemäß Fig. 9;
- Fig. 14: den schematischen Aufbau einer Vorrichtung zur Durchführung des Messverfahrens;
- Fig. 15: eine über die Zeit aufgetragene Positionsdarstellung unter Berücksichtigung des korrelierten Signals ähnlich Fig. 13, so genannter M-Mode;
- Fig. 16: eine Darstellung der Blutflussgeschwindigkeit mit Teilchenanzahl, so genannter Doppler.

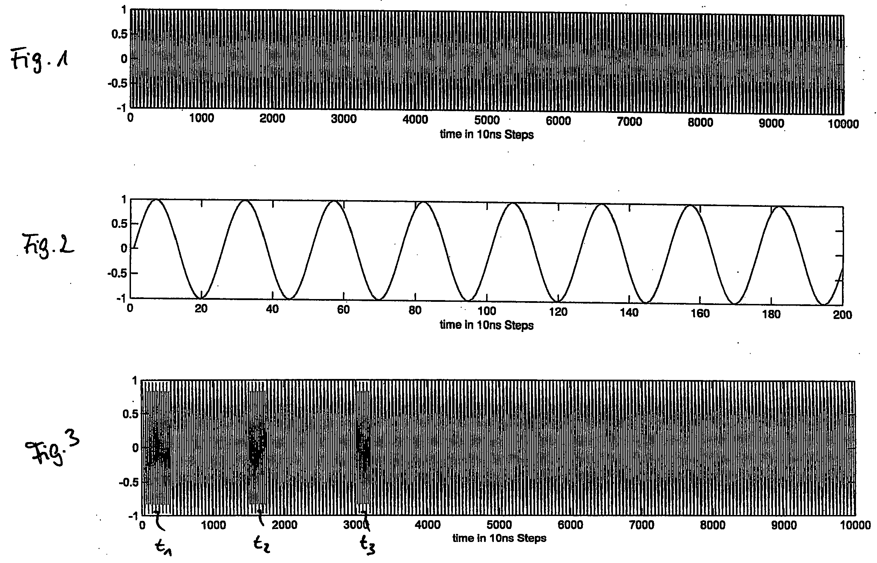

Fig. 1 zeigt ein Geschwindigkeitssignal mit konstanter Frequenz, beispielsweise zwei Megahertz. Dieses Signal entspricht im Prinzip dem Eingabesignal bei einer alleinigen Ultraschall-CW-Dopplermessung. Dieses Signal wird konstant fortlaufend kontinuierlich mit konstant bleibender Frequenz erzeugt.

Fig. 2 zeigt ein Positionssignal ebenfalls mit konstanter Frequenz, welches alternierend mit Pausenzeiten erzeugt wird. Die Frequenz liegt hier beispielsweise bei vier Megahertz. Ähnlich wie beim Ultraschall-PW-Messverfahren werden mit diesem Signal Pulse erzeugt und ausgesendet, beispielsweise Pulse einer Länge von 2 µs. Anhand der Zeit bis zur Detektion von Echos auf einen Puls kann Rückschluss auf den Reflektionspunkt gezogen werden, somit auf die Tiefe. Problematisch ist, dass für eine gute Auflösung kurze Wellenpakete erforderlich sind, welche wiederum eine geringe Anzahl Wellen enthalten. Dadurch wird aber beim herkömmlichen Ultraschall-PW-Verfahren die Genauigkeit der Geschwindigkeitsmessung über den Dopplereffekt schlechter.

Fig. 3 zeigt ein Mischsignal aus dem Geschwindigkeitssignal gemäß Fig. 1 und dem Positionssignal gemäß Fig. 2. Dabei ist in den Zeitbereichen t₁, t₂ und t₃ jeweils ein Puls gemäß Positionssignal aus Fig. 2 dem Geschwindigkeitssignal aus Fig. 1 beigemischt. Die mathematische Operation der Beimischung ist grundsätzlich beliebig.

Im Prinzip entspricht das Verfahren an dieser Stelle der gleichzeitigen Durchführung sowohl einer Ultraschall-CW-Messung als auch einer Ultraschall-PW-Messung.

Das Mischsignal wird über einen Sender in Richtung des zu untersuchenden Objekts bzw. des zu untersuchenden Mediums ausgesendet. Von dort wird das Mischsignal reflektiert und als Echosignal empfangen.

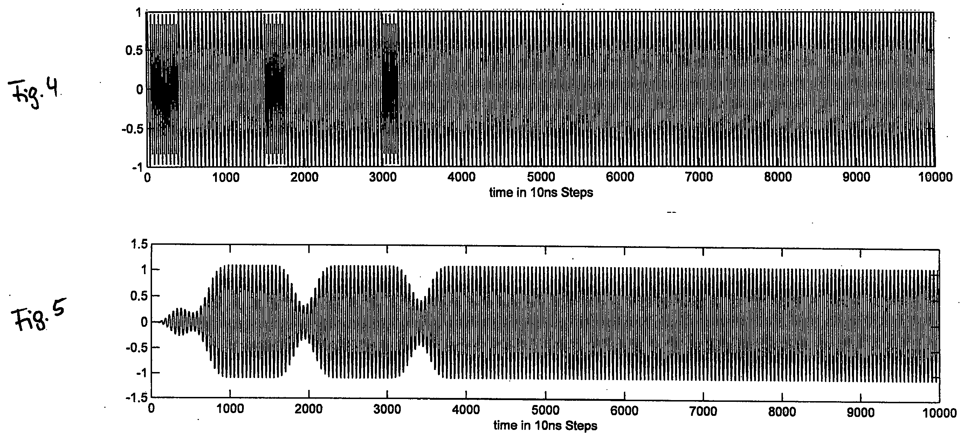

Das empfangene Echosignal ist in Fig. 4 dargestellt.

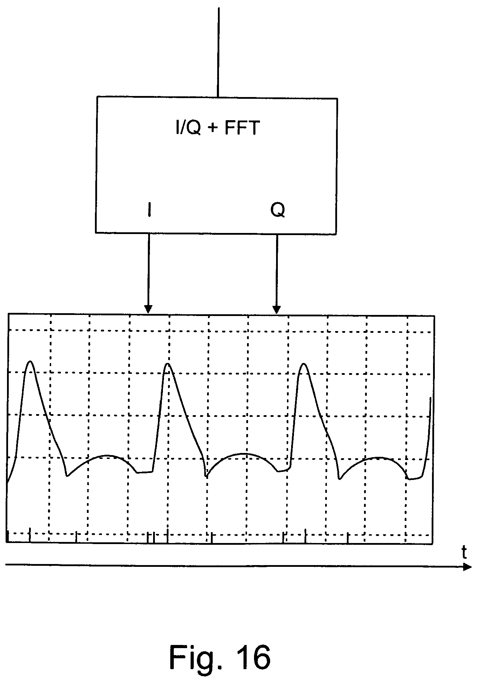

Fig. 5 zeigt das auf 2 Megahertz gefilterte Echosignal aus Fig. 4. 2 Megahertz entspricht nämlich genau der Frequenz des Geschwindigkeitssignals aus Fig. 1, über das die Geschwindigkeit bestimmt werden soll. Dieses gefilterte Echosignal wird sodann mit dem gesendeten Geschwindigkeitssignal aus Fig. 1 verglichen und einer FFT-Analyse unterzogen, um über die Frequenzveränderungen und den Dopplereffekt an die zu bestimmende Geschwindigkeit zu gelangen (siehe Darstellung Fig. 16). Somit wird die Geschwindigkeit kontinuierlich ohne Unterbrechungen bestimmt. Bei diesem Verfahren gibt es keine Totzeiten, zu denen die Geschwindigkeit unbekannt bleibt.

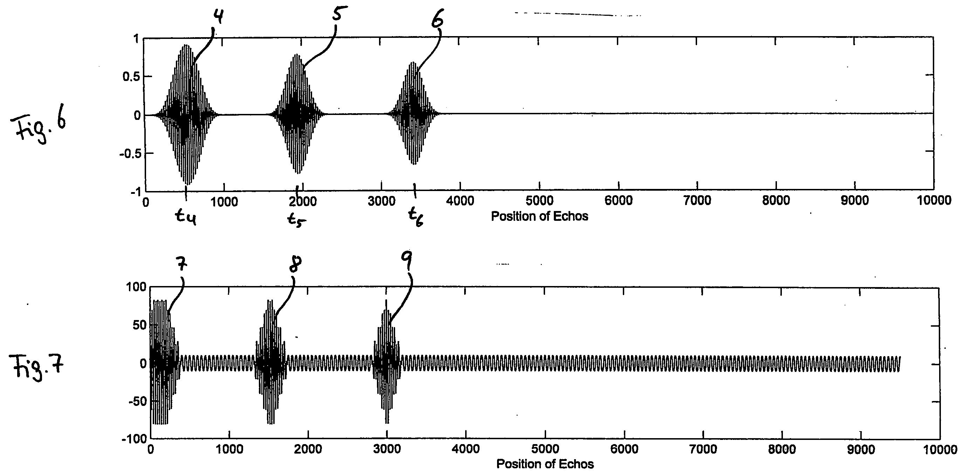

Fig. 6 zeigt das auf die Frequenz des Positionssignals aus Fig. 2, hier 4 Megahertz, gefilterte Echosignal aus Fig. 4. Erkennbar sind hier drei Echopakete 4, 5 und 6. Über die Echolaufzeiten t₄, t₅ und t₆ ist ein Rückschluss auf die Position bzw. Tiefenlage des reflektierenden Objekts bzw. Mediums möglich.

Weiterhin könnten aus den in den Echopaketen 4, 5 und 6 enthaltenen Dopplerinformationen Rückschlüsse auf die Geschwindigkeiten an den Reflektionsstellen gezogen werden, in der medizinischen Diagnostik könnte das Signal als Tissue-Doppler dargestellt werden. Dies bringt allerdings die erwähnten Nachteile mit sich, dass die Auflösung sehr beschränkt ist und die Messung diversen Totzeiträumen unterliegt.

Unabhängig davon, ob diese Dopplerauswertung aufgrund dieses Signals erfolgt oder nicht, wird in jedem Fall die hochgenaue Geschwindigkeitsinformation aus dem Signal gemäß Fig. 5 erhalten und weiterhin die Positionsinformation aus dem Signal gemäß Fig. 6. Somit sind kontinuierlich beide Größen mit einer Messung erhältlich.

Weiterhin ersichtlich ist auch eine mäßige Genauigkeit der Positionsbestimmung aufgrund dieses Signals, weil die Echopakete 4, 5 und 6 eine gewisse Breite aufweisen. Diese Breite ist insbesondere abhängig von der Länge der mit Signal 2 gemäß Fig. 2 gesendeten Wellenpakete.

Fig. 7 zeigt das Echosignal aus Fig. 4 korreliert mit dem Positionssignal aus Fig. 2.

Die Ähnlichkeit dieses Signals mit dem gefilterten Signal aus Fig. 6 ist ersichtlich. Allerdings wird sich an diesem korrelierten Signal keine Dopplerauswertung mehr durchführen lassen. Aufgrund der jedoch mit hoher Genauigkeit ermittelten Geschwindigkeit ist dies relativ unkritisch.

Der Rückschluss auf die Tiefenlage des Reflektionsortes lässt sich an diesem Signal genau wie an dem gefilterten Signal aus Fig. 6 vollziehen. Die Echopakete 7, 8 und 9 sind jedoch erkennbar schmaler als die Echopakete 4, 5 und 6 aus dem gefilterten Signal aus Fig. 6. Somit ist die Auflösung bei der Bestimmung der Tiefenlage bzw. Position der Reflektionsstelle bzw. des Mediums/Objekts bereits durch die Korrelation bei der Auswertung verbessert worden.

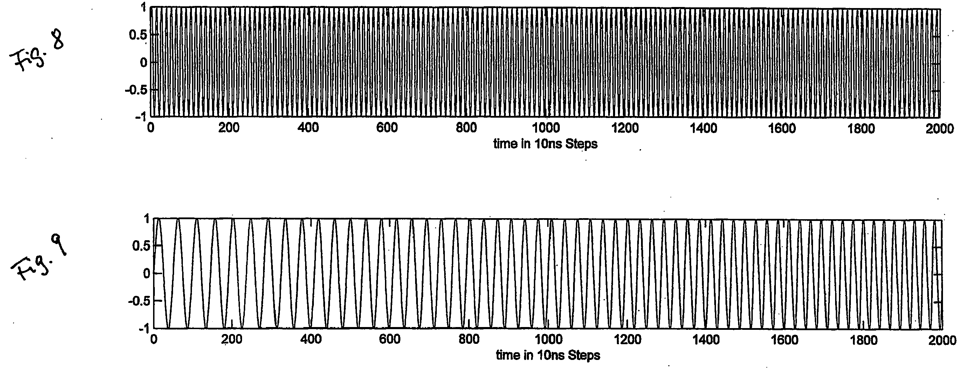

Fig. 8 zeigt ein Geschwindigkeitssignal gemäß einer zweiten Ausführungsform mit konstanter Frequenz von hier beispielsweise 8 Megahertz. Das Signal wird kontinuierlich ununterbrochen mit dieser Frequenz erzeugt und gesendet.

Fig. 9 zeigt ein Positionssignal gemäß der zweiten Ausführungsform. Das Signal kann kontinuierlich ununterbrochen erzeugt und gesendet werden, kann aber auch in Zeitintervallen alternierend mit einer Pause erzeugt bzw. gesendet werden.

In diesem Beispiel ist das Positionssignal frequenzmoduliert mit anfänglich zwei Megahertz mit linear ansteigend auf vier Megahertz und dann wieder linear abfallend auf zwei Megahertz. Nach der Erzeugung einer solchen Frequenzrampe wird das Signal zunächst für eine Pausenzeit ausgesetzt und danach wieder für die Dauer der Frequenzrampe erzeugt bzw. gesendet.

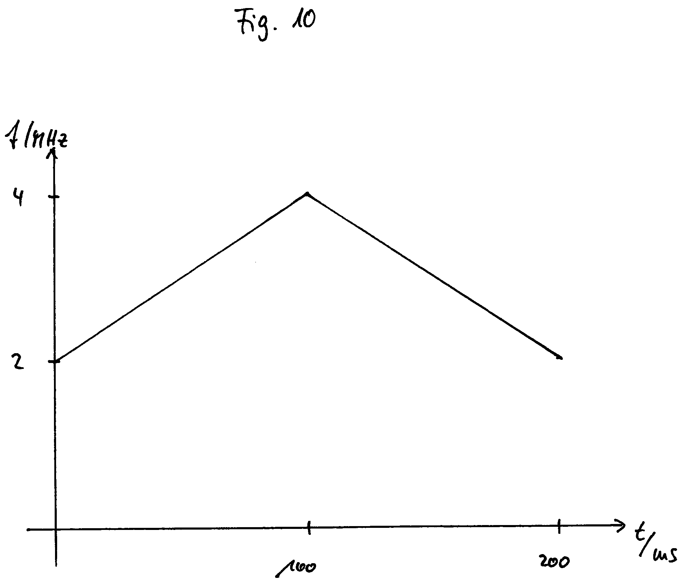

Fig. 10 zeigt die Frequenzmodulationsfunktion des Positionssignals aus Fig. 9. Dabei ist der lineare Anstieg von anfänglich zwei Megahertz auf vier Megahertz und zum Ende der Rampe hin ein Abfallen auf zwei Megahertz erkennbar. Grundsätzlich ist die Wahl der Modulationsfunktion beliebig. Schranken können beispielsweise durch mechanische Vorgaben bezüglich Reaktionsverzögerungen bei Piezokristallerzeugern auferlegt werden, was jedoch bei der Erzeugung von elektromagnetischen Wellen unproblematisch wäre.

Besonders vorteilhaft ist es, wenn die Frequenzmodulation dem Positionssignal eine eindeutige Kennzeichnung aufprägt. Dies schlägt sich nach der Korrelation in einer guten Auflösung nieder.

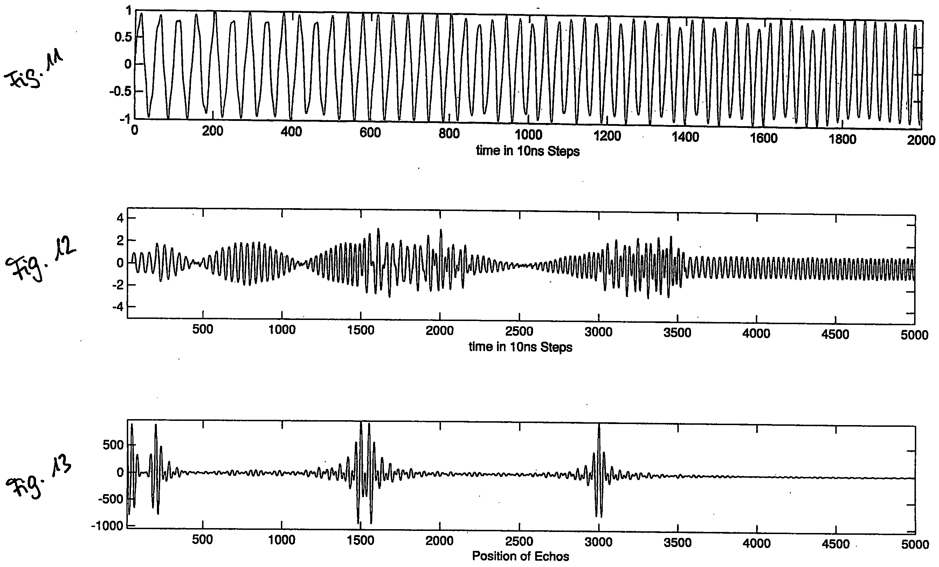

Fig. 11 zeigt ein Mischsignal aus Geschwindigkeitssignal nach Fig. 8 und Positionssignal nach Fig. 9. Dieses Mischsignal wird sodann an einen Sender geleitet, um wie bei der ersten Ausführungsform auch in Richtung des zu untersuchenden Mediums/Objektes gesendet zu werden.

Fig. 12 zeigt das nach Aussendung des Mischsignals gemäß Fig. 11 vom Medium/Objekt reflektierte Echosignal.

Die Auswertung mittels Dopplerverschiebung kann an diesem Signal - genau wie am Signal aus Fig. 4 - ggf. nach Filterung auf die Frequenz des Signals aus Fig. 8 - erfolgen, um an die Geschwindigkeit des Mediums/Objektes zu gelangen.

Fig. 13 zeigt das Echosignal aus Fig. 12 korreliert mit dem Positionssignal aus Fig. 9.

Die Auswertung von Position/Tiefe erfolgt an diesem Signal über die Laufzeit der Echopakete wie am Signal aus Fig. 7 der ersten Ausführungsform.

Deutlich erkennbar ist die geringere Bandbreite der reflektierten Echopakete verglichen mit dem Signal aus den Fig. 6 und 7. Damit ist die Auflösung und somit die Qualität der Positionsbestimmung bei dieser Ausführungsform erheblich verbessert. Deutlich erkennbar wird hier auch eine Trennung von eigentlich 2 Pulsen im Bereich von 0 bis ca. 250 und im Bereich um 1500, welche in den Signalen aus Fig. 6 und 7 noch nicht trennbar sind.

Die Auflösung ist abhängig von der Güte der Korrelation, somit also von der Eindeutigkeit der Kennzeichnung im Positionssignal. Je besser das Positionssignal korreliert, desto besser ist auch die Auflösung in diesem Signal.

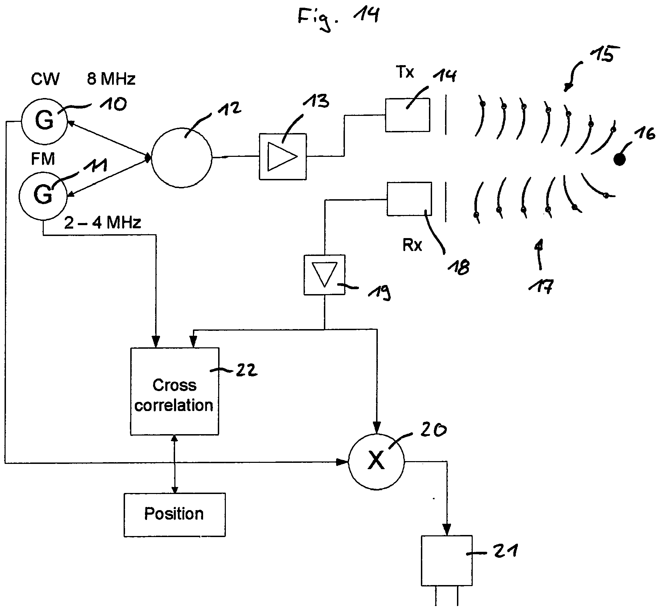

Fig. 14 zeigt den Aufbau einer Vorrichtung, mit der das Messverfahren der vorliegenden Erfindung durchgeführt werden kann. Der Geschwindigkeitssignalgenerator 10, beispielsweise ein CW-Signalgenerator, erzeugt ein Signal mit konstanter Frequenz von acht Megahertz nach Fig. 8. Der Positionssignalgenerator 11, beispielsweise ein FM-Signalgenerator, erzeugt ein Positionssignal mit von zwei bis vier Megahertz modulierter Frequenz nach Fig. 9. Beide Signale werden in einem Mischglied 12 vermischt und nach einer Verstärkung im Verstärker 13 an einen Sender 14 geleitet.

Der Sender 14 sendet die Schallwellen 15 in Richtung des zu untersuchenden Mediums/Objektes 16, an welchem die Schallwellen 15 eine Reflektion erfahren. Die reflektierten Schallwellen 17 gelangen dann zu einem Empfänger 18, der das empfangene Echosignal nach einer Verstärkung im Verstärker 19 zur Auswertung leitet.

Die Geschwindigkeitsauswertung findet unter Berücksichtigung des Geschwindigkeitssignals (vom Signalgenerator 10 erzeugt) in einem Mischer 20 statt, und die erhaltene Geschwindigkeitsinformation wird an ein Darstellungsglied 21 geleitet.

Die Positionsauswertung findet unter Berücksichtigung des vom Positionssignalgenerator 11 erzeugten Positionssignals in einem Korrelator 22 oder dergleichen statt. Im vorliegenden Beispiel erfolgt die Positionsauswertung unter anderem durch eine Kreuzkorrelation mit dem Positionssignal. Aus dem Ergebnissignal lässt sich, wie oben beschrieben, die Positionsinformation ableiten.

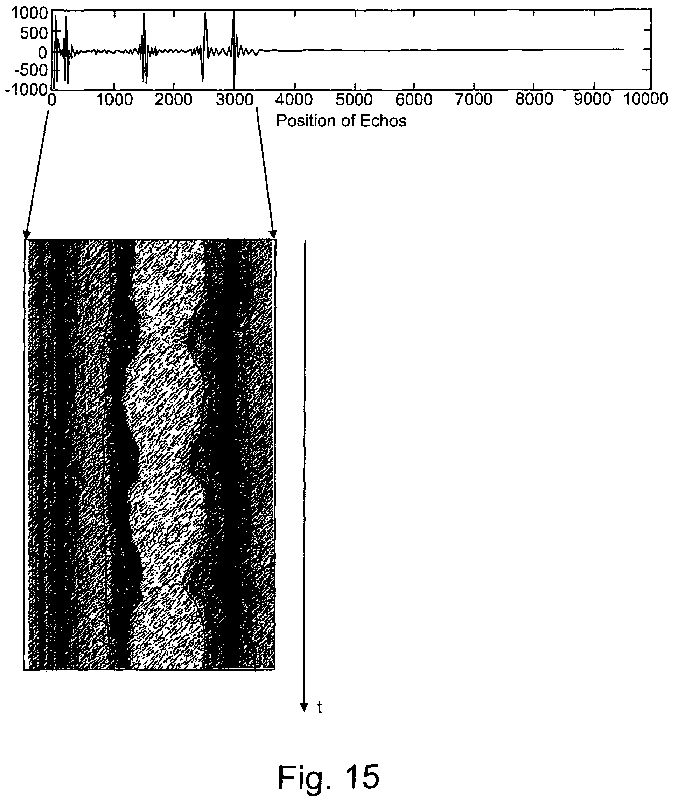

Fig. 15 zeigt eine Darstellungsmöglichkeit basierend auf einem Signal ähnlich Fig. 13. Bei der Untersuchung von beispielsweise pulsierenden Blutgefäßen wird diese Pulsation durch Veränderung der Laufzeiten der Echopakete sichtbar. Die Laufzeit der Echopakete und somit die Positionsinformation der Reflektionsstellen, wie beispielsweise der Gefäßwände, kann mit einer Bildhelligkeit versehen über die Zeit aufgetragen werden und in einem sog. M-Mode dargestellt werden. Damit erhält im Bereich der Medizintechnik der Behandler eine kontinuierliche Information über die Gefäßbreite bzw. Tiefe/Lage.

In Fig. 16 ist eine weitere Darstellung der aus der Geschwindigkeitsauswertung erhaltenen Geschwindigkeitsinformation gezeigt. Dabei wird die Geschwindigkeitsinformation beispielsweise nach Durchführung einer Fouriertransformation als Real- und Imaginärteil erhalten und über die Zeit aufgetragen dargestellt. In diesem Beispiel gibt die schwarze Linie die Anzahl der Teilchen und die Schattierung die Geschwindigkeiten der Teilchen wider.

Mit der vorliegenden Erfindung kann also simultan ohne Totzeiten sowohl die Geschwindigkeitsinformation als auch die Positionsinformation dargestellt werden. Damit kann ersehen werden, wie viele Teilchen wie schnell sind und in welcher Tiefe dieser Fluss zu vermuten ist. Es ist sogar eine Zuordnung des Flusses zu verschiedenen Blutgefäßen möglich, wenn sich beispielsweise im Messfeld mehrere Blutgefäße befinden.

Besondere Vorteile ergeben sich damit im Bereich der ultraschallgestützten Katheteruntersuchungen. Es ist nämlich mit der vorliegenden Erfindung möglich, gleichzeitig die herrschenden Blutflussbedingungen in den Blutgefäßen im Messfeld sowie Informationen über die Position eines Führungsdrahtes darzustellen. Damit erhält der Behandler kontinuierlich Informationen über Strömungsverhältnisse um die Katheterspitze und gleichzeitig die Lage des Katheters und kann deswegen frühzeitig einen Kollaps, beispielsweise über die Erkennung der Flusskurvenmuster und/oder Bestimmung der Reynoldszahl, erkennen und dem entgegen wirken.

## Patentansprüche

1. Verfahren zur simultanen Bestimmung von Position und Geschwindigkeit von Objekten (16), umfassend die Schritte:
- simultane Erzeugung eines ersten Signals (10) zur Geschwindigkeitsbestimmung und eines zweiten Signals (11) zur Positionsbestimmung,
- Mischen (12) des ersten Signals mit dem zweiten Signal zu einem Mischsignal und anschließendes Senden des Mischsignals mittels eines Senders (14) in Richtung eines Objektes, sodass das Mischsignal zumindest teilweise an dem Objekt reflektiert und als Echosignal empfangen wird,
- Auswertung des Echosignals,
**dadurch gekennzeichnet,**
**dass** die Auswertung umfasst:
- Durchführung einer Geschwindigkeitsauswertung des Echosignals unter Berücksichtigung des ersten Signals (10) und des Dopplereffektes, wobei als ein Ergebnis dieser Geschwindigkeitsauswertung eine Geschwindigkeitsinformation des Objektes erhalten wird,
- Durchführung einer Positionsauswertung des Echosignals unter Berücksichtigung des zweiten Signals (11), wobei als ein Ergebnis dieser Positionsauswertung eine Positionsinformation des Objektes erhalten wird ; wobei das Echosignal mit dem zweiten Signal zur Positionsbestimmung korreliert wird.

2. Verfahren nach Anspruch 1,
wobei das Verfahren weiterhin einen kontinuierlichen Empfang eines vom Objekt reflektierten Echosignals umfasst.

3. Verfahren nach Anspruch 1 oder 2,
wobei die Signale in Form von Ultraschallwellen gebildet sind.

4. Verfahren nach einem der Ansprüche 1 bis 3,
wobei die Signale in Form von elektromagnetischen Wellen gebildet sind.

5. Verfahren nach einem der Ansprüche 1 bis 4,
wobei das erste Signal ein dopplergünstiges Signal ist.

6. Verfahren nach einem der Ansprüche 1 bis 5,
wobei das zweite Signal von einem im Sinne der Mustererkennung positionsgünstigen Signaltyp ist.

7. Verfahren nach einem der Ansprüche 1 bis 6,
wobei das zweite Signal eine konstante Frequenz aufweist und in Intervallen gesendet wird.

8. Verfahren nach einem der Ansprüche 1 bis 7,
wobei das zweite Signal einer mathematisch korrelierbaren Funktion folgt.

9. Verfahren nach einem der Ansprüche 1 bis 8,
wobei das zweite Signal eine zeitlich modulierte Frequenz, FM, aufweist.

10. Verfahren nach einem der Ansprüche 1 bis 9,
wobei das Mischsignal einer im mathematischen Sinne glatten Funktion folgt.

11. Verfahren nach einem der Ansprüche 1 bis 10,
wobei die Positionsauswertung eine Filterung des Echosignals bezüglich der Frequenz des zweiten Signals umfasst.

12. Verfahren nach einem der Ansprüche 1 bis 11,
wobei die Positionsauswertung eine Analyse der Dopplerverschiebung und/oder der Echolaufzeit umfasst.

13. Verfahren nach einem der Ansprüche 1 bis 12,
wobei die Positionsauswertung eine Korrelation des Echosignals mit dem zweiten Signal umfasst und anschließend eine Analyse der Echolaufzeit durchgeführt wird.

14. Verwendung des Verfahrens nach einem der Ansprüche 1 bis 13 zur Überwachung der Zugangslegung in Blutgefäßen und/oder zur Perikardpunktion.

15. Verwendung des Verfahrens nach einem der Ansprüche 1 bis 13 zur Bestimmung von Blutflussgeschwindigkeit und Position von Blutgefäßen.

## Claims

1. Method for simultaneously determining the position and velocity of objects (16), said method comprising the steps of:
- simultaneously generating a first signal (10) for determining the velocity and a second signal (11) for determining the position,
- mixing (12) the first signal with the second signal to form a mixed signal and subsequently transmitting the mixed signal towards an object using a transmitter (14), so that the mixed signal can be at least partially reflected by the object and is received as an echo signal,
- evaluating the echo signal,
**characterised in that**
the evaluation comprises the steps of:
- performing a velocity evaluation of the echo signal by taking into account the first signal (10) and the Doppler effect, wherein velocity information as to the object is obtained as a result of said velocity evaluation,
- performing a position evaluation of the echo signal by taking into account the second signal (11), wherein positional information as to the object is obtained as a result of said position evaluation, wherein the echo signal is correlated (22) with the second signal for determining the position.

2. Method according to claim 1,
wherein the method further comprises the step of continuously receiving an echo signal reflected by the object.

3. Method according to claim 1 or 2,
wherein the signals are generated in the form of ultrasonic waves.

4. Method according to any of claims 1 to 3,
wherein the signals are generated in the form of electromagnetic waves.

5. Method according to any of claims 1 to 4,
wherein the first signal is of a signal type which is suitable in terms of the Doppler effect.

6. Method according to any of claims 1 to 5,
wherein the second signal is of a signal type which is suitably positioned in terms of pattern recognition.

7. Method according to any of claims 1 to 6,
wherein the second signal exhibits a constant frequency and is transmitted at intervals.

8. Method according to any of claims 1 to 7,
wherein the second signal obeys a function which can be mathematically correlated.

9. Method according to any of claims 1 to 8,
wherein the second signal exhibits a time-modulated frequency FM.

10. Method according to any of claims 1 to 9,
wherein the mixed signal obeys a mathematically smooth function.

11. Method according to any of claims 1 to 10,
wherein the position evaluation comprises filtering of the echo signal with respect to the frequency of the second signal.

12. Method according to any of claims 1 to 11,
wherein the position evaluation comprises an analysis of the Doppler shift and/or of the echo transmission time.

13. Method according to any of claims 1 to 12,
wherein the position evaluation comprises correlating the echo signal with the second signal and subsequently analysing the echo transmission time.

14. Use of the method according to any of claims 1 to 13 for monitoring blood vessel access and/or for pericardiocentesis.

15. Use of the method according to any of claims 1 to 13 for determining the blood flow velocity and the position of blood vessels.

## Revendications

1. Procédé de détermination simultanée de la position et de la vitesse d'objets (16), comprenant les étapes suivantes :
- générer simultanément un premier signal (10) destiné à déterminer la vitesse et un deuxième signal (11) destiné à déterminer la position,
- mélanger (12) le premier signal avec le deuxième signal, créant un signal de mélange, et transmettre ensuite le signal de mélange au moyen d'un transmetteur (14) vers un objet de manière que le signal de mélange est reflété au moins partiellement par l'objet et est reçu comme signal d'écho,
- évaluer le signal d'écho,
**caractérisé en ce que**
l'évaluation comprend les étapes suivantes :
- effectuer une évaluation de la vitesse du signal d'écho en tenant compte du premier signal (10) et de l'effet Doppler, une information sur la vitesse de l'objet étant obtenue comme résultat de cette évaluation de la vitesse,
- effectuer une évaluation de la position du signal d'écho en tenant compte du deuxième signal (11), une information sur la position de l'objet étant obtenue comme résultat de cette évaluation de la position, le signal d'écho étant corrélé avec le deuxième signal destiné à déterminer la position.

2. Procédé selon la revendication 1,
dans lequel le procédé comprend en outre la réception continue d'un signal d'écho reflété par l'objet.

3. Procédé selon la revendication 1 ou 2,
dans lequel les signaux sont générés sous la forme d'ondes ultrasonores.

4. Procédé selon l'une quelconque des revendications 1 à 3,
dans lequel les signaux sont générés sous la forme d'ondes électromagnétiques.

5. Procédé selon l'une quelconque des revendications 1 à 4,
dans lequel le premier signal est un type de signal qui est favorable en termes de l'effet Doppler.

6. Procédé selon l'une quelconque des revendications 1 à 5,
dans lequel le deuxième signal est un type de signal qui est positionné favorablement dans le sens de la reconnaissance de formes.

7. Procédé selon l'une quelconque des revendications 1 à 6,
dans lequel le deuxième signal a une fréquence constante et est transmis à intervalles.

8. Procédé selon l'une quelconque des revendications 1 à 7,
dans lequel le deuxième signal suit une fonction mathématiquement corrélable.

9. Procédé selon l'une quelconque des revendications 1 à 8,
dans lequel le deuxième signal a une fréquence FM modulée dans le temps.

10. Procédé selon l'une quelconque des revendications 1 à 9,
dans lequel le signal de mélange suit une fonction lisse dans le sens mathématique.

11. Procédé selon l'une quelconque des revendications 1 à 10,
dans lequel l'évaluation de la position comprend un filtrage du signal d'écho par rapport à la fréquence du deuxième signal.

12. Procédé selon l'une quelconque des revendications 1 à 11,
dans lequel l'évaluation de la position comprend une analyse du décalage Doppler et/ou du temps de propagation de l'écho.

13. Procédé selon l'une quelconque des revendications 1 à 12,
dans lequel l'évaluation de la position comprend une corrélation du signal d'écho avec le deuxième signal et ensuite le temps de propagation de l'écho est analysé.

14. Utilisation du procédé selon l'une quelconque des revendications 1 à 13 pour la surveillance de la mise en place d'une voie dans les vaisseaux sanguins et/ou pour la ponction péricardique.

15. Utilisation du procédé selon l'une quelconque des revendications 1 à 13 pour la détermination du débit sanguin et de la position des vaisseaux sanguins.
